(19) Europäisches Patentamt
European Patent Office
Office européen des brevets

(11) EP 1 790 238 A1

(12) EUROPEAN PATENT APPLICATION
published in accordance with Art. 158(3) EPC

(43) Date of publication:
**30.05.2007 Bulletin 2007/22**

(21) Application number: **05744119.8**

(22) Date of filing: **17.05.2005**

(51) Int Cl.:
***A23L 1/30*** *(2006.01)*

(86) International application number:
**PCT/JP2005/008968**

(87) International publication number:
**WO 2006/013665 (09.02.2006 Gazette 2006/06)**

(84) Designated Contracting States:
**AT BE BG CH CY CZ DE DK EE ES FI FR GB GR HU IE IS IT LI LT LU MC NL PL PT RO SE SI SK TR**

(30) Priority: **02.08.2004 JP 2004225051**
**30.11.2004 JP 2004346032**

(71) Applicant: **Kaneka Corporation**
**Kita-ku**
**Osaka-shi, Osaka 530-8288 (JP)**

(72) Inventors:
- **KITAMURA, Shiro,**
  **Hyogo 6730882; (JP)**
- **UEDA, Takahiro,**
  **Hyogo 6550872 (JP)**
- **UEDA, Yasuyoshi**
  **Hyogo 6711227 (JP)**
- **KISHIDA, Hideyuki,**
  **Hyogo 6750039 (JP)**
- **FUJII, Kenji,**
  **Hyogo 6511202, (JP)**
- **HOSOE, Kazunori,**
  **Hyogo 6760025 (JP)**

(74) Representative: **Hart Davis, Jason et al**
**Cabinet Beau de Loménie,**
**158, rue de l'Université**
**75340 Paris Cedex 07 (FR)**

# (54) WHITENING COMPOSITION CONTAINING REDUCED COENZYME Q

(57) The present invention provides a cosmetic or food composition which is excellent in a skincare effect, particularly an effect of whitening the skin browned due to ultraviolet radiation or aging, and which can be safely used for many mammals. The present invention provides a whitening composition comprising reduced coenzyme Q represented by formula (1) as an effective component: wherein n represents an integer of 1 to 12. The present invention also provides a whitening composition further comprising oxidized coenzyme Q represented by formula (2) as an effective component: wherein n represents an integer of 1 to 12. The present invention also provides a method for whitening the skin of a mammal, the method comprising administering the mammal with the composition.

EP 1 790 238 A1

## Description

### TECHNICAL FIELD

The present invention relates to a whitening composition containing reduced coenzyme Q. Also, the present invention relates to a method for producing the composition and a method for whitening the human skin with the composition. Reduced coenzyme Q is a compound useful as a food, Food with nutrient function claims, Food for specified health uses, a nutritious supplement, a nutritious agent, an animal drug, a drink, a feedstuff, a cosmetic, a medicine, a curative medicine, a preventive medicine, or the like. The whitening composition of the present invention has an excellent whitening ability and moisturizing ability, and the ability of preventing and improving skin roughness, preventing browning, preventing pigmentation, preventing flecking, improving wrinkles, preventing dullness, preventing pore-clogging dirt, and the like.

### BACKGROUND ART

Coenzyme Q is an essential component which distributes in a wide variety of living organisms ranging from bacteria to mammals, and is known as a component of the electron transport system of cellular mitochondria in a living body. It is also known that coenzyme Q repeats oxidation and reduction in mitochondria to have the function as a transport component in the electron transport system. Human coenzyme Q is mainly composed of coenzyme $Q_{10}$ having 10 repeat structures at its side chain. In a living body, oxidized coenzyme $Q_{10}$ and reduced coenzyme $Q_{10}$ exhibit a function while maintaining a balanced ratio therebetween, and about 40% to 90% of coenzyme Q present in a living body is generally a reduced type. Examples of a physiological function of coenzyme Q include the function to activate the energy production by a mitochondria activating action, the function to activate the cardiopulmonary function, the effect of stabilizing a cellular membrane, the effect of protecting cells by an antioxidative effect, and the like.

Although hydroquinone has been conventionally used as a substance having a whitening ability, it is undesirable because of its strong irritation to the skin and the possibility of allergic contact dermatitis as a side effect. In order to improve safety, therefore, an attempt has been made to form a monoester of a higher fatty acid, for example. However, such an ester is disadvantageous in safety because it is decomposed with a hydrolase in living organisms.

On the other hand, oxidized coenzyme $Q_{10}$ is widely used as a cosmetic component for improving wrinkles in Europe and the United States, and the safety thereof has been proved. Also, the inventors of the present invention have reported that reduced coenzyme $Q_{10}$ is a highly-safe substance without irritation to the skin.

Japanese Kohyo Publication Hei-09-501925 (Patent document 1) discloses a skin agent containing oxidized coenzyme $Q_{10}$ (ubiquinone) or reduced coenzyme $Q_{10}$ (ubiquinol) as coenzyme $Q_{10}$. However, this document discloses coenzyme $Q_{10}$ as only an example of the many components listed therein, and, in particular, an example of actual use of ubiquinol is not shown in the document. This document also discloses that the skin agent containing such coenzyme $Q_{10}$ is effective to various skin diseases. However, the whitening ability is not disclosed or suggested because of the absence of an example.

Japanese Kokai Publication 2001-085156 (Patent document 2) discloses that reduced coenzyme $Q_{10}$ has a higher curative action on atopic dermatitis than that of oxidized coenzyme $Q_{10}$. However, this document does not disclose or suggest the whitening ability.

Japanese Kokai Publication Sho-61-289029 (Patent document 3) discloses that ubiquinone (oxidized coenzyme Q) has the effect of suppressing chromatopathy, but ubiquinol is not mentioned. In addition, the specified chromatopathy is taken into consideration, but a relation to browning of the human skin due to ultraviolet light is not clarified at all.

Furthermore, widely used hydroquinone has strong irritation to the skin and thus has the possibility of allergic contact dermatitis as a side effect. Therefore, there has been demand for pharmaceutical, cosmetic, and food compositions having higher safety for the skin and the whitening ability or the ability of preventing pigmentation.

### SUMMARY OF THE INVENTION

Accordingly, it is an object of the present invention to provide a skin or food composition which is excellent in a skincare effect, particularly a whitening effect, and which can be safely used for many mammals, particularly humans.

As a result of intensive research for solving the above-described problem, the inventors of the present invention have found that a composition containing reduced coenzyme Q has an excellent whitening ability, resulting in the completion of the present invention.

The present invention provides the following:

[1] A whitening composition comprising reduced coenzyme Q represented by formula (1) as an effective component (wherein n represents an integer of 1 to 12).

[2] The composition described in [1] further comprising oxidized coenzyme Q represented by formula (2) as an effective component (wherein n represents an integer of 1 to 12).
[3] The composition described in [1] or [2], wherein n is 10.
[4] The composition described in [1] or [2] further comprising at least one antioxidant.
[5] The composition described in [4], wherein the antioxidant is selected from the group consisting of ascorbic acid and its derivatives, citric acid and its derivatives, pyrroloquinoline quinone and its derivatives, glutathione and its derivatives, vitamin E and its derivatives, polyphenols, catechins, carotinoids, pycnogenol, and flavangenol.
[6] The composition described in [5], wherein the antioxidant is selected from ascorbic acid and its derivatives.
[7] The composition described in [6], wherein the content of ascorbic acid is 0.01 part by weight to 50 parts by weight relative to 1 part by weight of coenzyme Q.
[8] The composition described in [1] or [2] further comprising at least one component selected from the group consisting of an ultraviolet absorber and an ultraviolet scattering agent.
[9] The composition described in [1] or [2] further comprising a moisturizing component.
[10] The composition described in [1] or [2] further comprising estrogen.
[11] The composition described in [1] or [2] further comprising vitamins.
[12] The composition described in [1] or [2] further comprising at least one selected from the group consisting of rucinol, placenta, placenta extract, allantoin, hydroquinone and its derivatives, hydroquinone glycosides, tranexamic acid and its derivatives, salicylic acid and its derivatives, kojic acid, ellagic acid, pantothenic acid, calcium pantothenate, menthyl lactate, resorcin and its derivatives, menthol derivatives, lipoic acid, arbutin, althea extract, licorice extract, Mori Cortex extract, raspberry extract, aloe, aloe extract, chamomile extract, g-oryzanol, flavonoid, herbal medicines and their extracts, and food materials and their extracts.
[13] The composition described in any one of [1] to [12] used for cosmetics.
[14] The composition described in any one of [1] to [12] used for foods.
[15] The composition described in any one of [1] to [12] used for medicines.
[16] A method for whitening the skin of a mammal, the method comprising administering the mammal with the composition described in any one of [1] to [15].
[17] A method for treating or preventing pigmentation in the skin of a mammal, the method comprising administering the mammal with the composition described in any one of [1] to [15] .
[18] A method for producing a whitening composition comprising mixing reduced coenzyme Q represented by formula (1) (wherein n represents an integer of 1 to 12) and a carrier, and, if required, oxidized coenzyme Q represented by formula (2) (wherein n represents an integer of 1 to 12) .

The present invention can provide a cosmetic composition having an excellent skincare effect, such as a whitening effect, and a food composition.

DETAILED DESCRIPTION OF THE INVENTION

The present invention will be described in detail below. Coenzyme Q is represented by formula (1):

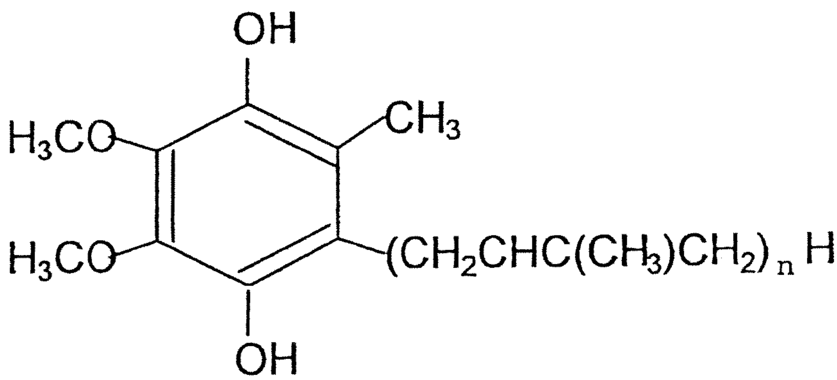

(1)

wherein n represents an integer of 1 to 12, or formula (2):

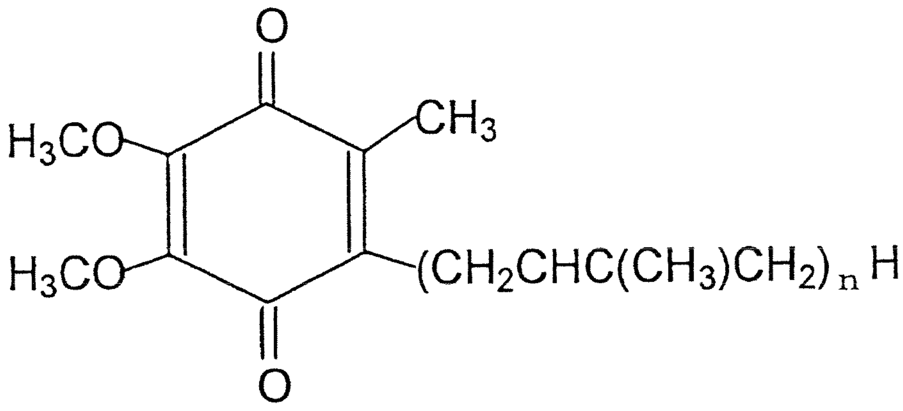

(2)

wherein n represents an integer of 1 to 12. Formula (1) represents reduced coenzyme Q, and formula (2) represents oxidized coenzyme Q.

A method for producing reduced coenzyme Q and oxidized coenzyme Q is not particularly limited. For example, coenzyme Q can be produced by a conventional known method, such as synthesis, fermentation, extraction from a natural substance, or the like. Alternatively, the coenzyme Q obtained as described above may be, for example, subjected to chromatography, and a fraction of oxidized coenzyme Q or reduced coenzyme Q of an eluate may be concentrated. When oxidized coenzyme Q is desired, the known method described in Japanese Kokai Publication Sho-52-72884 and the like can be used. In brief, oxidized coenzyme Q can be obtained by coupling 2,3-dimethoxy-5-methyl-1,4-benzohydroquinone to isoprenyl alcohol or its reactive derivative, and then oxidizing the product with an oxidizing agent, such as manganese dioxide.

When reduced coenzyme Q is desired, it can be produced by the known method described in Japanese Kokai Publication 2003-113129, and the like. In brief, a general reducing agent, such as ascorbic acid, sodium borohydride, or sodium dithionite (sodium hydrosulfite), is added to the coenzyme Q as occasion demands, and the oxidized coenzyme Q contained in the coenzyme Q is reduced to reduced coenzyme Q by an ordinary method, followed by concentration through chromatography. Alternatively, reduced coenzyme Q can be produced by a method of reacting existing high-purity coenzyme Q with the reducing agent.

The reduced coenzyme Q used in a whitening composition of the present invention is preferably reduced coenzyme $Q_{10}$ in which n is 10. Similarly, oxidized coenzyme $Q_{10}$ in which n is 10 is also preferred.

In an embodiment of the present invention, a whitening composition contains reduced coenzyme Q. In another embodiment of the present invention, a whitening composition may further contain oxidized coenzyme Q. The content of the reduced coenzyme Q in the total amount of the coenzyme Q is preferably 20% by weight or more, more preferably 50% by weight or more, further preferably 80% by weight or more, particularly preferably 90% by weight or more, and most preferably 95% by weight or more. The upper limit is 100% by weight and generally 99.9% by weight or less, but the upper limit is not particularly limited.

The content of the reduced coenzyme Q in the whitening composition of the present invention is not particularly limited, but the content is generally 0.001% by weight or more, preferably 0.01% by weight or more, and more preferably 0.1% by weight or more for improving the whitening effect. From an economical viewpoint, for example, the upper limit is, without limitation to, generally 10% by weight or less, preferably 5% by weight or less, and more preferably 1% by weight or less.

With respect to the amounts of the oxidized coenzyme Q and oxidized coenzyme Q, the amounts of the oxidized coenzyme Q and reduced coenzyme Q in a sample can be generally determined by a HPLC system using an UV detector. The amounts of the oxidized coenzyme Q and reduced coenzyme Q can be also determined by calculation from a peak area in a system in which an electrochemical detector is incorporated into HPLC. The system incorporated with the electrochemical detector is highly useful in measuring the trace amounts of oxidized coenzyme Q and reduced coenzyme Q present in a living organism or sample because the system can specifically measure oxidized and reduced substances and has high sensitivity. Specifically, the amounts are measured by a Shimadzu HPLC analyzer in which a Shimadzu UV detector or a Shiseido electrochemical detector is incorporated under the following conditions:

Column: YMC-Pack (ODS-A303)
Detection wavelength: 275 nm
Applied voltage: 600 mV (vs. Ag/AgCl)

Mobile phase: 88% methanol and 12% hexane (+50 mM $NaClO_4$)
Flow rate: 1 ml/min.

The term "whitening composition" means a composition capable of suppressing pigmentation, for example, melanin pigmentation, in the skin of a mammal. The whitening composition has the ability of whitening the human skin browned by pigmentation, for example, melanin pigmentation, in the skin of a mammal due to ultraviolet radiation or aging.
A method for producing the composition of the present invention is not particularly limited. For example, the composition can be produced by mixing the reduced coenzyme Q produced as described above and, if required, oxidized coenzyme Q, which is commercially available or produced by a known method, with the same carrier or respective carriers, and dissolving the resultant mixture if the mixture is soluble. Alternatively, the mixture of reduced coenzyme Q and oxidized coenzyme Q produced in the above-mentioned process for producing coenzyme Q may be directly mixed with a carrier. As the carrier, a carrier allowable in medicines, cosmetics, foods, and the like can be used according to demand within a range to an extent that the effect of the invention is not impaired.
Furthermore, the composition of the present invention can take various forms according to applications and purposes. For example, the composition can take any one of various forms, such as, but not limited to, a water-in-oil type emulsion composition, an oil-in-water type emulsion composition, and an oily composition.
The composition of the present invention can contain, as the carrier, any of the various components given below.
Examples of liquid oils and fats include avocado oil, camellia oil, jojoba oil, turtle oil, macadamia nut oil, corn oil, mink oil, rape oil, yolk oil, persic oil, wheat germ oil, Camellia sasanqua oil, castor oil, linseed oil, safflower oil, cottonseed oil, perilla oil, soybean oil, peanut oil, tea seed oil, kaya oil, rice bran oil, Chinese wood oil, Japanese wood oil, germ oil, and triglycerine.
Examples of solid oils and fats include cocoa butter, coconut oil, hydrogenated coconut oil, palm oil, palm kernel oil, Japan wax kernel oil, hydrogenated oil, Japan wax, and hydrogenated castor oil.
Examples of wax include beeswax, candelilla wax, cotton wax, carnauba wax, bayberry, Chinese wax, whale wax, montan wax, rice-bran wax, lanolin, kapok wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.
Examples of hydrocarbon oils include liquid paraffin, ozokerite, squalane, pristane, paraffin, ceresin, squalene, vaseline, and microcrystalline wax.
Examples of higher fatty acids include lauric acid, myristic acid, palmitic acid, stearic acid, behenic acid, oleic acid, undecylenic acid, tall oil fatty acid, isostearic acid, linoleic acid, linolenic acid, eicosapentaenoic acid (EPA), and docosahexaenoic acid (DHA) .
Examples of higher alcohols include linear alcohols, such as lauryl alcohol, cetyl alcohol, stearyl alcohol, behenyl alcohol, myristyl alcohol, oleyl alcohol, and cetostearyl alcohol; and branched alcohols, such as monostearyl glycerin ether (batyl alcohol), 2-decyltetradecynol, lanolin alcohol, cholesterol, phytosterol, hexyl dodecanol, isostearyl alcohol, and octyl dodecanol.
Examples of ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauryl-L-glutamic acid 2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.
Examples of silicone oils include linear polysiloxanes, such as dimethylpolysiloxane, methylphenylpolysiloxane, and diphenylpolysiloxane; cyclic polysiloxanes, such as octamethylcyclotetrasiloxane, decamethylcyclopentasiloxane, and dodecamethylcyclohexasiloxane; silicone resins having a three-dimensional network structure; silicone rubber; and various modified polysiloxanes, such as amino-modified polysiloxane, polyether-modified polysiloxane, alkyl-modified polysiloxane, and fluorine-modified polysiloxane.
Examples of a power component include inorganic powders, such as talc, kaoline, mica, cericite, muscovite, phlogopite, synthetic mica, red mica, biotite, vermiculite, magnesium carbonate, calcium carbonate, aluminum silicate, barium silicate, calcium silicate, magnesium silicate, strontium silicate, tungstic acid metal salts, magnesium, silica, zeolite, barium sulfate, calcined calcium sulfate (calcined gypsum), calcium phosphate, fluoroapatite, hydroxyapatite, ceramic powders, metal soaps (e.g., zinc myristate, calcium palmitate, and aluminum stearate), and boron nitride; organic

powders, such as polyamide resin powder (nylon powder), polyethylene powder, polymethyl methacrylate powder, polystyrene powder, powder of styrene-acrylic acid copolymer resin, benzoguanamine resin powder, polytetrafluoroethylene powder, and cellulose powder; inorganic white pigments, such as titanium dioxide and zinc oxide; inorganic red pigments, such as iron oxide (iron red) and iron titanate; inorganic blown pigments, such as iron oxide; inorganic yellow pigments, such as yellow iron oxide and ocher; inorganic black pigments, such as black iron oxide and low titanium oxide; inorganic violet pigments, such as manganese violet and cobalt violet; inorganic green pigments, such as chromium oxide, chromium hydroxide, and cobalt titanate; inorganic blue pigments, such as ultramarine blue pigment and iron blue pigment; pearl pigments, such as titanium oxide-coated mica, titanium oxide-coated bismuth oxychloride, titanium oxide-coated talc, colored titanium oxide-coated mica, bismuth oxychloride, and argentine; metal powder pigments, such as aluminum powder and copper powder; organic pigments, such as zirconium, barium, or aluminum lake (e.g., organic pigments, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments, such as chlorophyll and carotene.

Examples of an anionic surfactant include fatty acid soaps, such as sodium laurate and sodium palmitate; higher alkyl sulfates, such as sodium lauryl sulfate and potassium lauryl sulfate; alkyl ether sulfates, such as triethanolamine POE-lauryl sulfate and sodium POE-lauryl sulfate; N-acylsarcosinic acids, such as sodium lauroyl sacrosinate; higher fatty acid amidosulfonates, such as sodium N-myristoyl-N-methyltaurine, sodium palm oil fatty acid methyltauride, and sodium lauryl methyl tauride; phosphates, such as sodium POE-oleyl ether phosphate and POE-stearyl ether phosphate; sulfosuccinates, such as sodium di-2-ethylhexylsulfosuccinate, sodium monolauroyl monoethanolamide polyoxyethylene sulfosuccinate, and sodium laurylpolypropylene glycol sulfosuccinate; alkylbenzenesulfonates, such as sodium linear dodecylbenzenesulfonate, triethanolamine linear dodecylbenzenesulfonate, and linear dodecylbenzenesulfonic acid; higher fatty acid ester sulfates, such as sodium hydrogenated palm oil fatty acid glycerin sulfate; N-acylglutamates, such as sodium N-lauroylglutamate, disodium N-stearoylglutamate, and monosodium N-myristoyl-L-glutamate; sulfonated oils, such as Turkey red oil; POE-alkyl ether carboxylic acids; POE-alkyl allyl ether carboxylates; olefin sulfonates; higher fatty acid ester sulfonates; secondary alcohol sulfates; higher fatty acid alkylolamide sulfates; sodium lauroylmonoethanolamide succinate; ditriethanolamine N-palmitoylaspartate; and casein sodium.

Examples of a cationic surfactant include alkyl trimethyl ammonium salts, such as stearyl trimethyl ammonium chloride and lauryl trimethyl ammonium chloride; alkyl pyridinium salts, such as cetyl pyridinium chloride; distearyl dimethyl ammonium chloride dialkyl dimethyl ammonium salts; poly(N,N'-dimethyl-3,5-methylenepyridinium) chloride; alkyl quaternary ammonium salts; alkyl dimethyl benzyl ammonium salts; alkyl isoquinolinium salts; dialkyl morphonium salts; POE-alkylamines; alkyl amine salts; polyamine fatty acid derivatives; amyl alcohol fatty acid derivatives; benzalkonium chloride; and benzethonium chloride.

Examples of an ampholytic surfactant include imidazoline-type ampholytic surfactants, such as sodium 2-undecyl-N,N,N-(hydroxyethylcarboxymethyl)-2-imidazoline and 2-cocoyl-2-imidazoliniumhydroxide-1-carboxylethyloxy disodium salt; betaine-type surfactants, such as 2-heptadecyl-N-carboxymethyl-N-hydroxyethylimidazolinium betaine, betaine lauryldimethylaminoacetate, alkylbetaine, amidobetaine, and sulfobetaine.

Examples of a lipophilic nonionic surfactant include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acid esters, such as glycerin mono-cottonseed oil fatty acid ester, glycerin monoerucate, glycerin sesquioleate, glycerin monostearate, glycerin oleate pyroglutamate, and glycerin malate monostearate; propylene glycol fatty acid esters, such as propylene glycol monostearate; hydrogenated castor oil derivatives; and glycerin alkyl ethers.

Examples of a hydrophilic nonionic surfactant include POE-sorbitan fatty acid esters, such as POE-sorbitan monooleate, POE-sorbitan monostearate, POE-sorbitan monooleate, and POE-sorbitan tetraoleate; POE-sorbitol fatty acid esters, such as POE-sorbitol monolaurate, POE-sorbitol monooleate, POE-sorbitol pentaoleate, and POE-sorbitol monostearate; POE-glycerin fatty acid esters, such as POE-glycerin monostearate, POE-glycerin monoisostearate, POE-glycerin triisostearate, and POE-glycerin monooleate; POE-fatty acid esters, such as POE-distearate, POE-monodzoleate, and ethylene glycol distearate; POE-alkyl ethers, such as POE-lauryl ether, POE-oleyl ether, POE-stearyl ether, POE-behenyl ether, POE-2-octyldodecyl ether, and POE-cholestanol ether; Pluronic-type surfactants, such as Pluronic; POE/POP-alkyl ethers, such as POE/POP-cetyl ether, POE/POP-2-decyl tetradecyl ether, POE/POP-monobutyl ether, POE/POP-hydrogenated lanoline, and POE/POP-glycerin ether; tetra-POE/tetra-POP-ethylenediamine condensates, such as Tetronic; POE-caster oil/hydrogenated castor oil derivatives, such as POE-castor oil, POE-hydrogenated castor oil, POE-hydrogenated castor oil monoisostearate, POE-hydrogenated castor oil triisostearate, POE-hydrogenated castor oil monopyroglutamic monoisostearic acid diester, and POE-hydrogenated castor oil maleate; POE-beeswax lanoline derivatives, such as POE-sorbitol beeswax; alkanolamides, such as coconut oil fatty acid diethanolamide, lauric acid monoethanolamide, and fatty acid isopropanolamide; POE-propylene glycol fatty acid esters;

POE-alkylamines; POE-fatty acid amides; sucrose fatty acid esters; alkylethoxydimethylamine oxides; and trioleyl phosphate.

Examples of the moisturizing agent include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salts, dl-pyrrolidone carboxylates, short-chain soluble collagen, diglycerin (EO) PE adducts, chestnut rose extract, yarrow extract, and melilot extract.

Examples of a natural water-soluble polymer include vegetable polymers, such as gum arabic, tragacanth gum, galactan, guar gum, carob gum, karaya gum, carrageenan, pectin, agar, quince seed (marmelo), alga colloid (brown alga extract), starch (rice, corn, potato, and wheat), and glycyrrhizinic acid; microbial polymers, such as xanthan gum, dextran, succinoglucan, and pullulan; animal polymers, such as collagen, casein, albumin, and gelatin.

Examples of a semisynthetic water-soluble polymer include starch polymers, such as carboxymethyl starch and methylhydroxypropyl starch; cellulose polymers, such as methyl cellulose, ethyl cellulose, methylhydroxypropyl cellulose, hydroxyethyl cellulose, sodium cellulose sulfate, hydroxypropyl cellulose, carboxymethyl cellulose, sodium carboxymethyl cellulose, crystalline cellulose, and powdered cellulose; alginic acid polymers, such as sodium alginate and propylene glycol alginate.

Examples of a synthetic water-soluble polymer include vinyl polymers, such as polyvinyl alcohol, polyvinyl methyl ether, polyvinyl pyrrolidone, and carboxyvinyl polymer; polyoxyethylene polymers, such as polyoxyethylene/polyoxypropylene copolymers of polyethylene glycol 20,000, 40,000, or 60,000; acrylic polymers, such as sodium polyacrylate, polyethylacrylate, and polyacrylamide; polyethyleneimine; and cationic polymers.

Examples of a thickener include gum arabic, carrageenan, karaya gum, tragacanth gum, carob gum, quince seed (marmelo), casein, dextrin, gelatin, sodium pectate, sodium alginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarind gum, cellulose dialkyldimethylammonium sulfate, xanthan gum, aluminum magnesium silicate, bentonite, hectorite, AlMg silicate (Veegum), Laponite, silicic acid anhydride.

Examples of a sequestering agent include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediaminehydroxyethyl triacetate.

Examples of a lower alcohol include ethanol, proponol, isopropanol, isobutyl alcohol, and tert-butyl alcohol.

Examples of a polyhydric alcohol include dihydric alcohols, such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols, such as glycerin and trimethylolpropane; tetrahydric alcohols, such as pentaerythritol, e.g., 1,2,6-hexanetriol; pentahydric alcohols, such as xylitol; hexahydric alcohols, such as sorbitol and mannitol; polyhydric alcohols, such as diethylene glycol, dipropylene glycol, triethylene glycol, polypropylene glycol, tetraethylene glycol, diglycerin, polyethylene glycol, triglycerin, tetraglycerin, and polyglycerin; dihydric alcohol alkyl ethers, such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether; dihydric alcohol alkyl ethers, such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethy ether, propylene glycol monobutyl ether, propylene glycol isopropyl ether, dipropylene glycol methyl ether, dipropylene glycol ethyl ether, and dipropylene glycol butyl ether; dihydric alcohol ether esters, such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate; glycerin monoalkyl ethers, such as chimyl alcohol, selachyl alcohol, and batyl alcohol; sugar alcohols, such as sorbitol, maltitol, maltotriose, mannitol, sucrose, erythritol, glucose, fructose, amylolytic sugars, maltose, xylitose, and reduced alcohols of amylolytic sugars; glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphate; POP/POE-pentaerythritol ether; and polyglycerin.

Examples of a monosaccharide include trioses, such as D-glycerin aldehyde and dihydroxyacetone; tetroses, such as D-erythrose, D-erythrulose, D-threose, and erythritol; pentoses, such as L-arabinose, D-xylose, L-lyxose, D-arabinose, D-ribose, D-ribulose, D-xylulose, and L-xylulose; hexoses, such as D-glucose, D-talose, D-psicose, D-galactose, D-fructose, L-galactose, L-mannose, and D-tagatose; heptoses, such as aldoheptose and heprose; octoses, such as octulose; deoxy sugars, such as 2-deoxy-D-ribose, 6-deoxy-L-galactose, and 6-deoxy-L-mannose; amino sugars, such as D-glucosamine, D-galactosamine, sialic acid, aminouronic acid, and muramic acid; uronic acids, such as D-glucuronic acid, D-mannuronic acid, L-guluronic acid, D-galacturonic acid, and L-iduronic acid.

Examples of oligosaccharides include sucrose, umbelliferose, lactose, planteose, isolignoses, trehalose, raffinose, lignoses, umbilicine, stachyose, and verbascose.

Examples of polysaccharides include cellulose, quince seed, chondroitin sulfate, starch, galactan, dermatan sulfate, glycogen, gum arabic, heparin sulfate, hyaluronic acid, tragacanth gum, keratan sulfate, chondroitin, xanthan gum, mucoitin sulfate, guar gum, dextran, keratosulfate, locust bean gum, succinoglucan, and charonic acid.

Examples of amino acids include neutral amino acids, such as threonine and systeine; and basic amino acids, such as hydroxylysine. Examples of amino acid derivatives include sodium acylsarcosinate (sodium laurylsarcosinate), acylglumatic acid salts, sodium acyl-b-alanine, glutathione, and pyrrolidone carboxylic acid.

Examples of organic amines such as monoethanolamine, diethanolamine, triethanolamine, morpholine, triisopropanolamine, 2-amino-2-methyl-1,3-propandiol, and 2-amino-2-methyl-1-propanol.

Examples of a polymer emulsion include an acrylic resin emulsion, a polyethyl acrylate emulsion, an acrylic resin solution, a polyalkyl acrylate emulsion, a polyvinyl acetate resin emulsion, and a natural rubber latex.

Examples of a pH adjuster include buffers, such as lactic acid-sodium lactate, citric acid-sodium citrate, and succinic acid-sodium succinate.

Examples of vitamins include vitamins A, B1, B2, B6, C, and E, and derivatives thereof; pantothenic acid and its derivatives; and biotin.

Examples of an antioxidant include ascorbic acid (vitamin C) and its derivatives, sodium hydrogen sulfite, sodium thiosulfate, sodium pyrosulfite, citric acid and its derivatives, tocopherols (vitamin E and its derivatives), glutathione and its derivatives, dibutylhydroxytoluene, butylated hydroxyanisole, gallic acid esters, pyrroloquinoline quinone and its derivatives, astaxanthin and its derivatives, polyphenols, catechins, pycnogenol, and flavangenol. Among these compounds, ascorbic acid and its derivatives, citric acid its derivatives, pyrroloquinoline quinone and its derivatives, astaxanthin and its derivatives, glutathione and its derivatives, vitamin E and its derivatives, polyphenols, catechins, pycnogenol, and flavangenol are preferred.

In particular, ascorbic acid is preferred because of safety for the skin. When ascorbic acid is used, the content of ascorbic acid is preferably 0.01 part by weight to 50 parts by weight and more preferably 0.1 part by weight to 5 parts by weight relative to 1 part by weight of coenzyme Q (total of oxidized form and reduced form).

Examples of an antioxidation auxiliary include phosphoric acid, citric acid, ascorbic acid, maleic acid, malonic acid, succinic acid, fumaric acid, cephalin, hexametaphosphate, phytic acid, and ethylenediaminetetraacetic acid.

Examples of the ultraviolet absorber include benzoic-acid-based ultraviolet absorbers, anthranilic-acid-based ultraviolet absorbers, salicylic-acid-based ultraviolet absorbers, cinnamic-acid-based ultraviolet absorbers, and triazine-based ultraviolet absorbers.

Examples of the benzoic-acid-based ultraviolet absorbers include paraaminobenzoic acid (referred to as "PABA" hereinafter), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester.

Examples of the anthranilic-acid-based ultraviolet absorbers include homomenthyl-N-acetylanthranilate.

Examples of the salicylic-acid-based ultraviolet absorbers include amyl salicylate, menthyl salicylate, homomenthyl salicylate, octyl salicylate, phenyl salicylate, benzyl salicylate, and p-isopropanolphenyl salicylate.

Examples of the cinnamic-acid-based ultraviolet absorbers include octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethylcyanophenyl cinnamate, 2-ethylhexylcyanophenyl cinnamate, and glyceryl-mono-2-ethylhexanoyl-diparamethoxy cinnamate.

An example of the triazine-based ultraviolet absorbers is bisresorcinyltriazine. Specific examples include bis{[4-(2-ethylhexyloxy)-2-hydoxy]phenyl}-6-(4-methoxyphenyl)-1,3,5-trizaine and 2,4,6-tris {4-(2-ethylhexyloxycarbonyl)anilino}-1,3,5-trizaine.

Other examples of an ultraviolet absorber include 3-(4'-methylbenzilidene)-d,l-camphor, 3-benzilidene-d,l-camphor, 2-phenyl-5-methylbenzoxazole, 2,2'-hydroxy-5-methylphenylbenzotrlazole, 2-(2'-hydroxy-5'-tert-octylphenyl)benzotriazole, 2-(2'-hydroxy-5'-methylphenyl)benzotriazole, dibenzalazine, dianisoylmethane, 4-methoxy-4'-tert-butyldibenzoylmethane, and 5-(3,3-dimethyl-2-norbornylidene)-3-pentan-2-one.

Among these ultraviolet absorbers, octylmethoxy cinnamate, decamethylcyclopentasiloxane, methylpolysiloxane, and dimethicone copolyol are preferred.

Examples of other components which can be compounded include preservatives, such as methylparaben, ethylparaben, and butylparaben; antiphlogistic agents, such as glycyrrhizinic acid derivatives, glycyrrhetinic acid derivatives, salicylic acid derivatives, hinokitiol, zinc oxide, and allantoin; whitening agents, such as strawberry geranium extract and arbutin; various extracts, such as extracts of cork tree bark, goldthread, Shikon (lithospermi radix), peony root, sialid, birch, sage, Japan medlar, carrot, aloe, mallow, iris, grape, Yokuinin (coicis semen), sponge gourd, lily,

saffron, Senkyu (cnidii rhizoma), Shokyo (rhizoma), rose of Sharon, ononis spinosa, garlic, pepper, citrus peel, Toki (angelicae radix), and seaweed; activator agents, such as royal jelly, photosensitive elements, and cholesterol derivatives; hematogenic agents, such as benzyl nicotinate, butoxyethyl nicotinate, capsaicin, zingerone, canthris tincture, ichthammol, tannic acid, borneol, tocophenol nicotinate, inositol hexanicotinate, cyclandelate, cinnarizine, tolazoline, acetylcholine, verapamil, sepharanthin, and oryzanol; and antiseborrheic agents, such as sulfur and thiantol; and anti-inflammatory agents, such as tranexamic acid, thiotaurine, and hypotaurine.

The whitening composition can contain the ultraviolet scattering agent, for example, titanium oxide or zinc oxide.

The composition of the present invention can contain a moisturizing component. Examples of the moisturizing component include hyaluronic acid, glycerin, urea, collagen, hydrolytic collagen, placenta extract, royal jelly, and lactoferrin.

The composition can further contain estrogen, for example, ethynyl estradiol or soybean isoflavone.

The composition can further contain rucinol or its derivative, hydroquinone or its derivative, a hydroquinone glycoside, tranexamic acid or its derivative, salicylic acid or its derivative, kojic acid, ellagic acid, menthyl lactate, resorcin or its derivative, a menthol derivative, lipoic acid, a herbal medicine or its extract, and/or a food material or its extract.

Preferred examples of an oily cosmetic of the present invention include a cosmetic oil, a skincare oil, a cleansing oil, and a bath oil.

The composition of the present invention may further contain an appropriate material other than the coenzyme Q. Examples of such a material include, but not limited to, an excipient, a disintegrator, a lubricant, a binder, a colorant, an aggregation inhibitor, and an absorption accelerator.

Examples of the excipient include, but not limited to, sucrose, lactose, glucose, corn starch, mannitol, crystalline cellulose, calcium phosphate, and calcium sulfate.

Examples of the disintegrator include, but not limited to, starch, agar, calcium citrate, calcium carbonate, sodium hydrogen carbonate, dextrin, crystalline cellulose, carboxymethyl cellulose, and tragacanth.

Examples of the lubricant include, but not limited to, talc, magnesium stearate, polyethylene glycol, silica, and hydrogenated vegetable oil.

Examples of the binder include, but not limited to, ethyl cellulose, methyl cellulose, hydroxypropylmethyl cellulose, tragacanth, shellac, gelatin, gum arabic, polyvinylpyrrolidone, polyvinyl alcohol, polyacrylic acid, polymethacrylic acid, and sorbitol.

The colorant is not particular limited, but, for example, colorants allowable to be added to medicines and foods can be used.

Examples of the aggregation inhibitor include, but not limited to, stearic acid, talc, light anhydrous silicic acid, and hydrous silicon dioxide.

Examples of the absorption accelerator include, but not limited to, surfactants, such as higher alcohols, higher fatty acids, sucrose fatty acid esters and sorbitan fatty acid esters, polyoxyethylene sorbitan fatty acid esters, and polyglycerin fatty acid esters. From the viewpoint of the stability of reduced coenzyme Q, polyglycerin fatty acid esters are particularly preferred. In particular, the lower limit of HLB (Hydrophilic-Lipophilic Balance) is generally 4 or more, preferably 5 or more, more preferably 6 or more, further preferably 7 or more, and particularly preferably 8 or more. The upper limit of HLB is generally 12 or less, preferably 11 or less, and more preferably 10 or less.

Preferred examples of the polyglycerin fatty acid esters include diglycerin monocuprate, diglycerin monolaurate, tetraglycerin monolaurate, glycerin monooleate, diglycerin dioleate, tetraglycerin monooleate, and decaglycerin pentaoleate. Among these, diglycerin monolaurate and diglycerin monooleate are more preferred, and diglycerin monooleate is most preferred.

Examples of a solubilizing agent for the above-described effective components include, but not limited to, organic acids such as fumaric acid, succinic acid, and malic acid.

Examples of the stabilizer include, but not limited to, benzoic acid, sodium benzoate, and ethyl paraoxybenzoate.

The composition of the present invention may further contain an active component other than the reduced coenzyme Q. Examples of such an active component includes amino acids, vitamins, minerals, polyphenols, organic acids, saccharides, peptides, and proteins.

When the absorption accelerator, particularly a polyglycerin fatty acid ester, among the above-described carriers is added, the bioabsorbability of the reduced coenzyme Q can be increased, and the antioxidation function of the composition of the present invention can be effectively exhibited.

The term "derivative" means a compound produced by slightly changing the structure of a compound. Namely, a compound produced by substituting a hydrogen atom or a specified atom group of an original compound with another atom or atom group is understood as a derivative of the original compound. Specifically, ascorbic acid derivatives include, but not limited to, ascorbyl palmitate, sodium ascorbate, ascorbyl stearate, and erythorbic acid. Citric acid derivatives include, but not limited to, sodium citrate, disodium citrate, sodium dihydrogen citrate, isopropyl citrate, calcium citrate, and triethyl citrate. Pyrroloquinoline quinone derivatives include, but not limited to, sodium pyrroloquinoline quinone. Glutathione derivatives include, but not limited to, oxidized glutathione. Vitamin E derivatives include, but not limited to,

tocopherol acetate, a-tocopherol, and b-tocopherol. Hydroquinone derivatives include, but not limited to, a-arbutin and b-arbutin. Tranexamic acid derivatives include, but not limited to, tranexamic acid amide. Salicylic acid derivatives include, but not limited to, ethylene glycol salicylate, sodium salicylate, phenyl salicylate, methyl salicylate, and lipohydroxylic acid. Resorcin derivatives include, but not limited to, rucinol. Menthol derivatives include, but not limited to, dl-menthol, 1-menthol, and menthyl lactate. Carotenoids include, but not limited to, b-carotin, a-carotin, lycopene, lutein, and astaxanthin. Polyphenols include, but not limited to, tannin, flavonoid, isoflavone, anthocyanin, and pycnogenol. Catechins include, but not limited to, catechin, epicatechin, epigallocatechin, gallocatechin, and theaflavin.

The composition containing the reduced coenzyme Q can be used as it is, but the composition can be preferably used as an oral formulation such as a capsule (hard capsule, soft capsule, or mlcrocapsule), a tablet, syrup, or a drink. Among these formulations, a capsule is particularly preferred, and a soft capsule is most preferred.

Usable examples of a capsule base material include, but not limited to, gelatin derived from beef bones, cow hide, pig hide, and fish skin etc.; and other base materials usable as food additives, such as seaweed-derived materials, e.g., carrageenan and alginic acid, vegetable-seed-derived materials, e.g., locust bean gum and guar gum, microbial materials, e. g., pullulan and curdlan, and manufacturing agents, e.g., celluloses.

The composition of the present invention can be used for medicines.

The composition of the present invention can also be used for cosmetics because the composition has a whitening ability. Examples of the form of a final cosmetic product include, but not limited to, an ointment, a lotion, a skin lotion, a cream, a non-sheet material impregnated with a cosmetic (mask), and a pack.

The composition of the present invention can be also used for foods. Also, the composition of the present invention can be contained in a food, Food with nutrient function claims, Food for specified health uses, a nutritious supplement, a trophic agent, a drink, a feedstuff, a medicine, a curative, a preventive, and a general food, i.e., an obvious food. For example, the composition can be appropriately added to bread, pasta, porridge, rice, biscuits, crackers, cakes, sweets, gums, and candies etc. Of course, the composition can be used in another food form.

The method for adding the reduced coenzyme Q to the composition for any one of the above-described foods, medicines, and cosmetics is not particularly limited. The reduced coenzyme Q may be added in the production process of these products, or the composition containing the reduced coenzyme Q prepared separately may be added to the final products.

Examples of administration include enteral administration, such as oral administration and transrectal administration; non-enteral administration, such as dermal administration, transmucosal administration, hypodermic administration, intravenous administration, and intraperitoneal administration. Examples of mammals include humans.

## BEST MODE FOR CARRYING OUT THE INVENTION

The present invention will be described in detail below with reference to examples, but the present invention is not limited to these examples.

## REFERENCE EXAMPLE

In 1000 g of ethanol, 100 g of oxidized coenzyme $Q_{10}$ and 60 g of L-ascorbic acid were added, and the resultant mixture was stirred at 78°C to effect reduction reaction. Thirty hours after, the mixture was cooled to 50°C, and 400 g of ethanol and 100 g of water were added to the mixture kept at the same temperature. Then, the resultant ethanol solution was cooled to 2°C at a cooling rate of 10 °C/hr under stirring, and then washed with cold ethanol and cold water in order. The resultant wet crystals were dried under reduced pressure to obtain 95 g of dry white crystals (effective yield 95 mol%). All operations excluding drying under reduced pressure were performed in a nitrogen atmosphere. The ratio by weight of reduced coenzyme $Q_{10}$/oxidized coenzyme $Q_{10}$ in the resultant crystals was 99.5/0.5.

## EXAMPLE 1

The reduced coenzyme $Q_{10}$ prepared in REFERENCE EXAMPLE was added to a mixture of rice oil, hydrogenated oil, beeswax, lecithin, and diglycerin monooleate, and a gelatin soft capsule formulation containing the components below was prepared by an ordinary method.

| | |
|---|---|
| Reduced coenzyme $Q_{10}$ | 60 parts by weight |
| Rice oil | 510 parts by weight |
| Diglycerin monooleate | 180 parts by weight |
| Hydrogenated oil | 170 parts by weight |
| Beeswax | 60 parts by weight |

(continued)

| | |
|---|---|
| Lecithin | 20 parts by weight |

EXAMPLE 2

The reduced coenzyme $Q_{10}$ prepared in REFERENCE EXAMPLE was dissolved in propanol, and the resultant solution was adsorbed on microcrystalline cellulose, followed by drying under reduced pressure. The cellulose was mixed with corn starch, lactose, carboxymethyl cellulose, and magnesium stearate in a nitrogen atmosphere, and then an aqueous solution of polyvinylpyrrolidone was added as a binder to the mixture. Then, the resultant mixture was granulated by an ordinary method, and talc was added as a lubricant to and mixed with the granules, followed by tableting.

| | |
|---|---|
| Reduced coenzyme $Q_{10}$ | 160 parts by weight |
| Corn starch | 200 parts by weight |
| Lactose | 120 parts by weight |
| Carboxylmethyl cellulose | 80 parts by weight |
| Microcrystalline cellulose | 300 parts by weight |
| Polyvinylpyrrolidone | 40 parts by weight |
| Magnesium stearate | 20 parts by weight |
| Talc | 80 parts by weight |

EXAMPLE 3

A cream having the composition below containing the reduced coenzyme $Q_{10}$ prepared in REFERENCE EXAMPLE was prepared by an ordinary method.

| | |
|---|---|
| Reduced coenzyme $Q_{10}$ | 10 parts by weight |
| Glycerol sorbitan fatty acid ester | 60 parts by weight |
| Microcrystalline wax | 10 parts by weight |
| Olive oil | 30 parts by weight |
| Liquid paraffin | 180 parts by weight |
| Magnesium stearate | 10 parts by weight |
| Propylene glycol | 37 parts by weight |
| Magnesium sulfate | 7 parts by weight |
| Dehydrated salt | 655 parts by weight |

EXAMPLE 4

A cream having the composition below containing the reduced coenzyme $Q_{10}$ prepared in REFERENCE EXAMPLE was prepared by an ordinary method.

| | |
|---|---|
| Reduced coenzyme $Q_{10}$ | 0.1 parts by weight |
| Glycerol sorbitan fatty acid ester | 60 parts by weight |
| Microcrystalline wax | 10 parts by weight |
| Olive oil | 40 parts by weight |
| Liquid paraffin | 180 parts by weight |
| Magnesium stearate | 10 parts by weight |
| Propylene glycol | 37 parts by weight |
| Magnesium sulfate | 7 parts by weight |
| Dehydrated salt | 655 parts by weight |

EXAMPLE 5

Inhibitory effect on pigmentation due to ultraviolet radiation

The dorsal hair of six brown guinea pigs was carefully shaved with a clipper or shaver, and the shaved dorsal skin of each guinea pig was irradiated with ultraviolet beams (UVB: wavelength 250 to 350 nm) in the minimum erythema dose at two square portions of 2 cm ' 2 cm on the right and left of the dorsal median. The ultraviolet irradiation was performed once every other day for three days to induce erythema. Also, a medium and a sample were continuously applied to the respective irradiated portions (erythema portions) once a day for one month to examine the inhibitory action on pigmentation. As the sample, a polyethylene glycol ointment containing 10 parts by weight of reduced coenzyme $Q_{10}$ was used. As the medium, only polyethylene glycol was used. Evaluation was performed by measurement with a color-difference meter, and an L value was calculated from the observed Munsell value.

Namely, DL (change with time) of the medium-applied portion was subtracted from DL (change with time) of the opposite sample-applied portion to obtain a value (#) according to the following equation:

$$\# = (L1 - L0) - (L'1 - L'0)$$

L0: Test portion before application of the sample
L'0: Test portion before application of the medium
L1: Sample-applied test portion one month after continuous application
L'1: Medium-applied test portion one month after continuous application

An evaluation result is shown by an average of evaluation scores (Table 1) of the six test animals. The results shown in Table 2 indicate that a composition containing reduced coenzyme $Q_{10}$ shows an evaluation score of 2 or more and thus has an inhibitory action on pigmented spots.

Table 1

| Evaluation score | Criteria |
|---|---|
| 5 | $2.0 \leq \#$ |
| 4 | $1.0 \leq \# < 2.0$ |
| 3 | $0.5 \leq \# < 1.0$ |
| 2 | $0 \leq \# < 0.5$ |
| 1 | $\# < 0$ |

Table 2

| Evaluated sample | Average evaluation score |
|---|---|
| Reduced coenzyme $Q_{10}$ | 2.17 |

## Claims

**1.** A whitening composition comprising reduced coenzyme Q represented by formula (1) as an effective component:

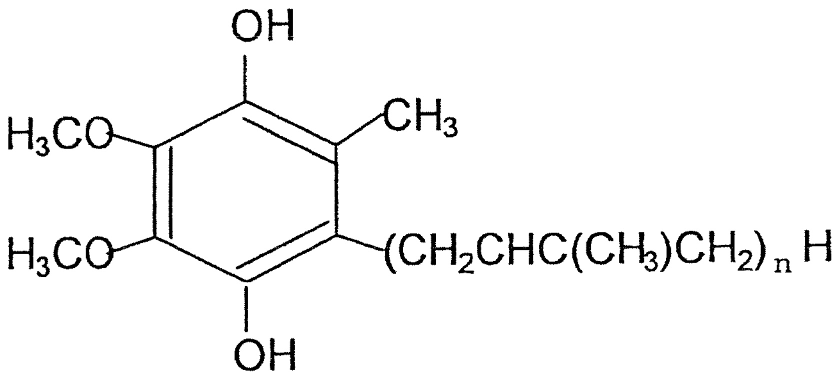

(1)

wherein n represents an integer of 1 to 12.

**2.** The composition according to claim 1, further comprising oxidized coenzyme Q represented by formula (2) as an effective component:

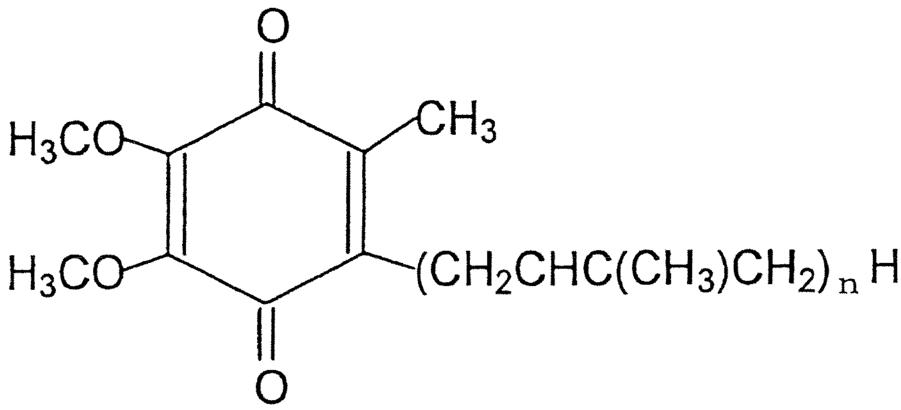

(2)

wherein n represents an integer of 1 to 12.

**3.** The composition according to claim 1 or 2, wherein n is 10.

**4.** The composition according to claim 1 or 2, further comprising at least one antioxidant.

**5.** The composition according to claim 4, wherein the antioxidant is selected from the group consisting of ascorbic acid and its derivatives, citric acid and its derivatives, pyrroloquinoline quinone and its derivatives, glutathione and its derivatives, vitamin E and its derivatives, polyphenols, catechins, carotinoids, pycnogenol, and flavangenol.

**6.** The composition according to claim 5, wherein the antioxidant is selected from ascorbic acid and its derivatives.

**7.** The composition according to claim 6, wherein the content of ascorbic acid is 0.01 part by weight to 50 parts by weight relative to 1 part by weight of coenzyme Q.

**8.** The composition according to claim 1 or 2, further comprising at least one component selected from the group consisting of an ultraviolet absorber and an ultraviolet scattering agent.

**9.** The composition according to claim 1 or 2, further comprising a moisturizing component.

**10.** The composition according to claim 1 or 2, further comprising estrogen.

**11.** The composition according to claim 1 or 2, further comprising vitamins.

**12.** The composition according to claim 1 or 2, further comprising at least one selected from the group consisting of rucinol, placenta, placenta extract, allantoin, hydroquinone and its derivatives, hydroquinone glycosides, tranexamic acid and its derivatives, salicylic acid and its derivatives, kojic acid, ellagic acid, pantothenic acid, calcium pantothenate, menthyl lactate, resorcin and its derivatives, menthol derivatives, lipoic acid, arbutin, althea extract, licorice extract, Mori Cortex extract, raspberry extract, aloe, aloe extract, chamomile extract, g-oryzanol, flavonoid, herbal medicines and their extracts, and food materials and their extracts.

**13.** The composition according to any one of claims 1 to 12 used for cosmetics.

**14.** The composition according to any one of claims 1 to 12 used for foods.

**15.** The composition according to any one of claims 1 to 12 used for medicines.

**16.** A method for whitening the skin of a mammal, the method comprising administering the mammal with the composition according to any one of claims 1 to 15.

**17.** A method for treating or preventing pigmentation in the skin of a mammal, the method comprising administering the mammal with the composition according to any one of claims 1 to 15.

**18.** A method for producing a whitening composition comprising mixing reduced coenzyme Q represented by formula (1) (wherein n represents an integer of 1 to 12) and a carrier, and, if required, oxidized coenzyme Q represented by formula (2) (wherein n represents an integer of 1 to 12).

## INTERNATIONAL SEARCH REPORT

International application No.
PCT/JP2005/008968

A. CLASSIFICATION OF SUBJECT MATTER
Int.Cl$^7$ A61K7/48, A23L1/30, A61K7/00

According to International Patent Classification (IPC) or to both national classification and IPC

B. FIELDS SEARCHED

Minimum documentation searched (classification system followed by classification symbols)
Int.Cl$^7$ A61K7/48, A23L1/30, A61K7/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| X/<br><br>Y | JP 61-27909 A (Shiseido Co., Ltd.), 07 February, 1986 (07.02.86), Full text (Family: none) | 1,3,4,8-13, 15,18/<br>2,5-7,14 |
| Y | WO 01/85156 A (KANEKA Corp.), 15 November, 2001 (15.11.01), Full text & EP 1281398 A1 | 1-15,18 |
| Y | WO 98/7417 A (KANEKA Corp.), 26 February, 1998 (26.02.98), Full text & EP 956854 A1 & JP 10-109933 A | 1-15,18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June, 2005 (16.06.05) | 05 July, 2005 (05.07.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/008968

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-107262 A (Nikko Chemicals Kabushiki Kaisha),<br>08 April, 2004 (08.04.04),<br>Full text<br>(Family: none) | 1-15,18 |
| Y | JP 2003-277219 A (Sukoyaka Shokuhin Kabushiki Kaisha),<br>02 October, 2003 (02.10.03),<br>Full text<br>(Family: none) | 1-15,18 |
| Y | EP 211183 A2 (Eisai Co., Ltd.),<br>25 February, 1987 (25.02.87),<br>Full text<br>& JP 61-289029 A | 1-15,18 |
| Y | JP 2003-238386 A (Yugen Kaisha Nonogawa Shoji),<br>27 August, 2003 (27.08.03),<br>Full text<br>(Family: none) | 1-15,18 |
| Y | WO 99/56719 A1 (GS Development A.B.),<br>11 November, 1999 (11.11.99),<br>Full text<br>& JP 2002-513746 A | 1-15,18 |
| Y | EP 1243252 A1 (L' oreal),<br>25 September, 2002 (25.09.02),<br>Full text<br>& JP 2002-322049 A | 1-15,18 |
| Y | US 6048886 A (USA),<br>11 April, 2000 (11.04.00),<br>Full text<br>(Family: none) | 1-15,18 |

Form PCT/ISA/210 (continuation of second sheet) (January 2004)

INTERNATIONAL SEARCH REPORT

International application No.
PCT/JP2005/008968

**Box No. II Observations where certain claims were found unsearchable (Continuation of item 2 of first sheet)**

This international search report has not been established in respect of certain claims under Article 17(2)(a) for the following reasons:

1. [X] Claims Nos.: 16, 17
because they relate to subject matter not required to be searched by this Authority, namely:
Claims 16 and 17 involve methods for treatment of the human body by surgery or therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9501925 A
- JP 2001085156 A
- JP 61289029 A
- JP 52072884 A
- JP 2003113129 A